(19) Europäisches Patentamt
European Patent Office
Office européen des brevets

(11) Veröffentlichungsnummer: **0 321 408 B1**

(12) **EUROPÄISCHE PATENTSCHRIFT**

(45) Veröffentlichungstag der Patentschrift: **23.09.92**

(51) Int. Cl.5: **C07D 285/12**, C07D 417/12, A01N 43/82

(21) Anmeldenummer: **88810852.9**

(22) Anmeldetag: **09.12.88**

Die Akte enthält technische Angaben, die nach dem Eingang der Anmeldung eingereicht wurden und die nicht in dieser Patentschrift enthalten sind.

# (54) 2-Thio-5-difluoromethylthio-1,3,4-thiadiazole und diese enthaltende nematizide Mittel.

(30) Priorität: **18.12.87 CH 4955/87**

(43) Veröffentlichungstag der Anmeldung:
**21.06.89 Patentblatt 89/25**

(45) Bekanntmachung des Hinweises auf die Patenterteilung:
**23.09.92 Patentblatt 92/39**

(84) Benannte Vertragsstaaten:
**AT BE CH DE ES FR GB GR IT LI LU NL**

(56) Entgegenhaltungen:
**EP-A- 0 217 747**
**WO-A-86/07590**

(73) Patentinhaber: **CIBA-GEIGY AG**
**Klybeckstrasse 141**
**CH-4002 Basel(CH)**

(72) Erfinder: **Beriger, Ernst, Dr.**
**Grabenmattweg 29**
**CH-4123 Allschwil(CH)**
Erfinder: **Eckhardt, Wolfgang, Dr.**
**Breslauerstrasse 14**
**W-7850 Lörrach(DE)**

**EP 0 321 408 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte 2-Thio-5-difluormethylthio-1,3,4-thiadiazole, deren Herstellung sowie nematizide Mittel, die als Wirkstoff mindestens eine dieser Verbindungen enthalten. Die Erfindung betrifft ferner die Verwendung von 2-Thio-5-difluormethylthio-1,3,4-thiadiazolen und Mittel zur Bekämpfung von Nematoden, insbesondere von pflanzenschädigenden Nematoden.

Die erfindungsgemässen 2-Thio-5-difluormethylthio-1,3,4-thiadiazole entsprechen der Formel I

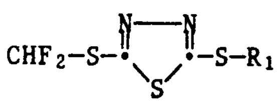

(I)

worin

$R_1$ unsubstituiertes oder durch Halogen, Phenyl, $C_1$-$C_3$-Alkoxy oder Hydroxy substituiertes $C_1$-$C_{12}$-Alkyl oder durch Carbonyl, O-Alkylcarboxyl, Amino oder durch einen Heterocyclus substituiertes $C_1$-$C_3$-Alkyl, unsubstituiertes oder durch Halogen substituiertes $C_3$-$C_7$-Cycloalkyl oder unsubstituiertes Cyclopropylmethyl, unsubstituiertes oder durch Halogen substituiertes $C_3$-$C_7$-Alkenyl, unsubstituiertes oder durch Halogen substituiertes $C_3$-$C_7$-Alkinyl, unsubstituiertes oder durch $C_1$-$C_4$-Alkyl, Halogen, Nitro, Cyano oder Trifluormethyl ein-, zwei- oder dreifach kernsubstituiertes Benzyl oder den Rest $R_2$-(O-$CH_2$-$CH_2$-$)_n$, bedeutet, worin $R_2$ für Wasserstoff, oder für unsubstituiertes oder durch Hydroxy substituiertes $C_1$-$C_4$-Alkyl und n für die Zahlen 1-3 stehen.

Unter Alkyl sind als selbständiger Rest sowie als Teil einer anderen Gruppe, wie Alkoxy, O-Alkylcarboxyl oder gerad- und verzweigtkettige Alkylgruppen zu verstehen. Dazu zählen die Methyl-, die Ethyl- sowie die normalen und isomeren Propyl-, Butyl- und Pentylgruppen. Halogensubstituiertes Alkyl steht für einen einfach bis perhalogenierten Alkylrest, wie z.B. $CHCl_2$, $CH_2F$, $CCl_3$, $CH_2Cl$; $CHFCH_3$, $CH_2CH_2Br$, $CF_2CF_3$, $C_2Cl_5$, $CH_2Br$, CHBrCl usw., vorzugsweise für $CHF_2$. Alkenyl bedeutet z.B. Propenyl-(1), Allyl, Butenyl-(1), Butenyl-(2) oder Butenyl-(3) sowie Ketten mit mehreren Doppelbindungen. Alkinyl bedeutet z.B. Propinyl-(2), Butinyl-(1), Butinyl-(2), Pentinyl-(4) usw.. Halogen steht für Fluor, Chlor, Brom oder Jod, vorzugsweise für Fluor, Chlor oder Brom.

Als Cycloalkyl sind Cyclopropyl, Cyclopentyl, Cyclohexyl und Cycloheptyl zu verstehen. Zu den Heterocyclen zählen Furan, Thiophen, Oxazol und Oxadiazol.

Es sind bereits Thiadiazol-Derivate bekannt, die als nematizid wirksam beschrieben sind. So sind in der US-Patentschrift 3,770,754 solche Verbindungen mit einer 1,2,4-Stellung der Heteroatome offenbart, während in der US-Patentschrift 4,454,147 1,3,4-Thiadiazol-Derivate beschrieben sind, bei denen im Vergleich mit den erfindungsgemässen Verbindungen der Heterocyclus anstelle der Mercapto-Gruppen durch ein Chloratom substituiert ist. Ferner sind aus der EP-A 217 747 auch 5-Phenyl-2-fluoralkyl-1,3,4-thiadiazole mit nematizider Wirkung bekannt, und in der WO 86/07590 (PCT) ist die Verbindung der Formel V

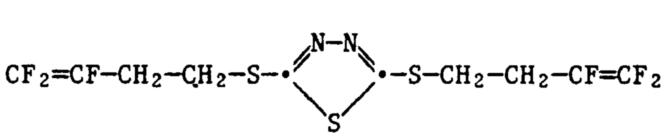

(V)

als Nematizid beschrieben.

Diese bekannten Verbindungen haben bisher als Nematizide die in der Praxis an sie gestellten Ansprüche nicht in vollem Umfang befriedigen können. Die erfindungsgemässen Verbindungen der Formel I haben einen für Bodenapplikation günstigen Verteilungskoeffizienten (log P) sowie genügend hohe Wasserlöslichkeit.

Durch die Bereitstellung der erfindungsgemässen Verbindungen der Formel I ist es nun gelungen einen wertvollen Beitrag zur Bekämpfung der beträchtliche landwirtschaftliche Schäden an Pflanzgut verursachenden Pflanzennematoden zu leisten. Auf diese Weise können Ernteeinbussen bei Kulturpflanzen wie z.B. Kartoffeln, Getreide, Rüben, Raps, Kohl, Tabak, Sojabohnen, Baumwolle und Gemüse sowie Schäden in Baumschulen und im Zierpflanzenbau nachhaltig eingedämmt werden. Die erfindungsgemässen Verbindungen zeichnen sich dabei insbesondere in der wirkungsvollen Bekämpfung von wurzelparasitären Bodennematoden wie z.B. solchen der Gattungen Heterodera und Globodera (zystenbildende Nematoden), Meloidogyne (Wurzelgallennematoden) sowie der Gattungen Radopholus, Pratylenchus, Tylenchulus, Longidorus, Trichodorus und Xiphinema aus. Ferner lassen sich mit den erfindungsgemässen Wirkstoffen die Nematodengattungen Ditylenchus (Stengelparasiten) Aphelenchoides (Blattnematoden) und Anguina (Blütennematoden) wirkungsvoll bekämpfen.

Mit den Wirkstoffen der Formel I lassen sich vorzugsweise besonders schädliche Nematodenarten der Gattung Meloidogyne, wie z.B. Meloidogyne incognita, sowie der Gattung Heterodera, wie z.B. Heterodera glycines (Sojabohnen-Zystennematode) und ferner der Gattung Globodera, wie z.B. Globodera rostochiensis (Kartoffelzystennematode) sowie Vertreter von wandernden Endoparasiten, wie z.B. Pratylenchus penetrans oder Radopholus similis und Vertreter von Ektoparasiten, wie z.B. Trichodorus spp. und Xiphinema spp. erfolgreich bekämpfen.

Zur Bekämpfung der Pflanzennematoden und zur Gesunderhaltung des Pflanzgutes können die neuen Wirkstoffe kurativ, präventiv oder systemisch eingesetzt werden. Dabei entfalten sie eine breit gefächerte Aktivität gegen die verschiedenen Nematodenspecies und werden somit den Erfordernissen der Praxis gerecht. Die nematizide Wirkungsweise der erfindungsgemässen Verbindungen wird durch eine geringe Phytotoxizität in vorteilhafter Weise begleitet, womit der allgemein wünschenswerten Verminderung der Umweltbelastung in besonderem Masse Rechnung getragen wird.

Im Rahmen der vorliegenden Erfindung sind jene 2-Thio-5-difluormethylthio-1,3,4-thiadiazole bevorzugt, bei welchen $R_1$ unsubstituiertes oder durch Halogen, Phenyl, $C_1$-$C_3$-Alkoxy oder Hydroxy substituiertes $C_1$-$C_4$-Alkyl, $C_3$-$C_4$-Alkenyl, $C_3$-$C_4$-Alkinyl, ferner den Carbomethoxy-methyl-, den Acetonyl-, den 2-Dimethylamino-äthyl-, den 2-Furanylmethyl-, den 2-Thienylmethyl-, den 3-Methyl-oxazol-5-yl-methyl-, den 5-Methyl-1,2,4-oxadiazol-3-yl-methylrest oder den Rest

$$R_2-(OCH_2CH_2)_n-$$

bedeutet.

Insbesondere bevorzugt sind von dieser Gruppe jene 2-Thio-5-difluormethylthio-1,3,4-thiadiazole, in welchen $R_1$ unsubstituiertes oder durch Halogen substituiertes $C_1$-$C_3$-Alkyl, ferner $C_3$-Alkenyl, $C_3$-Alkinyl und unsubstituiertes oder durch Halogen ein-oder zweifach kernsubstituiertes Benzyl bedeutet.

Von dieser Gruppe ist das 2,5-Bis(difluormethylthio)-1,3,4-thiadiazol als eine der wichtigen Einzelverbindungen hervorzuheben.

Verbindungen der Formel I werden erfindungsgemäss hergestellt, indem man

a) das 2,5-Dimercapto-1,3,4-thiadiazol der Formel II in einem Lösungsmittel zunächst mit einer Halogenverbindung der Formel III, worin $R_1$ die im Anspruch 1 angegebene Bedeutung hat und X für Chlor oder Brom steht, in Gegenwart einer Base zum 5-Mercapto-1,3,4-thiadiazol-Derivat der Formel IV umsetzt, dann das resultierende Zwischenprodukt der Formel IV in einem Lösungsmittel mit Difluorchlormethan in Gegenwart einer Base in das Endprodukt der Formel I überführt:

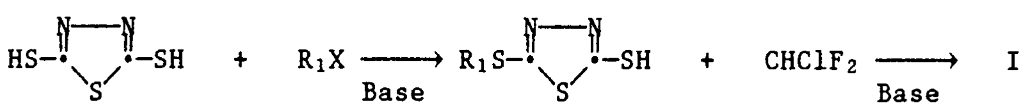


II III IV

b) eine Verbindung der Formel VI in einem Lösungsmittel mit Difluorchlormethan zum Endprodukt der Formel I in Analogie zu der in der Zeitschrift für anorganische und allgemeine Chemie (533 (1986) 99-108) beschriebenen Methode umsetzt:

$$K_2(S_2C=N-NH-CS_2R) + 2CHClF_2 \longrightarrow I.$$

VI

Für die Herstellung des Zwischenproduktes der Formel IV nach der Variante a) eignen sich als Lösungsmittel aliphatische und aromatische Kohlenwasserstoffe wie Benzol, Toluol, Petrolether, Alkohole wie z.B. Methanol, Ethanol, Isopropanol oder Butanol, Ketone wie Aceton, Diethylketon, Methylethylketon, Ether und etherartige Verbindungen wie Dialkylether (Diethylether, Diisopropylether, tert.-Butylmethylether

usw.), Anisol, Dioxan, Tetrahydrofuran, aliphatische und aromatische halogenierte Kohlenwasserstoffe wie Chlorbenzol, Methylenchlorid, Chloroform, Ethylenchlorid, Tetrachlorkohlenstoff, Tetrachlorethylen und ganz allgemein Gemische solcher Lösungsmittel untereinander.

Für diese Stufe kommen als Basen organische und anorganische Basen in Betracht; z.B. Ammoniak, Amine, wie Triethylamin, Triallylamin; sowie Hydroxide von Alkali- und Erdalkalimetallen, wie NaOH, KOH, $Ca(OH)_2$,

Für die Herstellung der erfindungsgemässen Wirksubstanz aus dem Zwischenprodukt der Formel IV geeignete Lösungs- oder Verdünnungsmittel sind z.B. Ether und etherartige Verbindungen wie Dialkylether, Dioxan, Tetrahydrofuran, gegebenenfalls als wässrige Mischungen.

Als Basen für diese Stufe kommen anorganische Basen in Betracht, sowie Oxide, Hydroxide, Carbonate und Hydrogencarbonate von Alkali-und Erdalkalimetallen, vorzugsweise NaOH und KOH.

Für die Herstellung der erfindungsgemässen Wirksubstanzen nach Variante b) eignen sich polare Lösungsmittel wie Alkohole, ferner Dimethylformamid oder Dimethylsulfoxid.

In den Herstellungsverfahren betragen die Reaktionstemperaturen 0 bis 90 °C, vorzugsweise 20° bis 60 °C. Für die Druckverhältnisse während des Reaktionsablaufs sind 1 bis 20 bar, vorzugsweise 1 bar, massgebend.

Die Ausgangsverbindungen der Formel II bzw. V sind bekannt, J. Org. Chem. 21 497-499, (1956) bzw. Acta Chem. Scan. 15 1295-1302 (1961).

Die Erfindung betrifft auch Mittel, zur Bekämpfung von pflanzenschädigenden Nematoden sowie zur präventiven Verhütung des Nematodenbefalls von Pflanzgut, welche die Wirkstoffe der Formel I enthalten.

Darüber hinaus schliesst die vorliegende Erfindung zusätzlich die Herstellung nematizider Mittel ein, die gekennzeichnet ist durch das innige Vermischen von Aktivsubstanzen der Formel I mit einer oder mehreren hierin beschriebenen Trägerstoffen und Hilfsingredienzen.

Eingeschlossen ist auch ein Verfahren zur Behandlung von Pflanzen, das durch die Applikation der Verbindungen der Formel I oder der neuen Mittel charakterisiert ist.

Ein bezvorzugtes Verfahren für den Einsatz eines Wirkstoffs der Formel I bzw. eines nematiziden Mittels, das mindestens einen dieser Wirkstoffe enthält, ist das Einbringen in den Erdboden. Dabei wird der Standort der Pflanzen mit einer flüssigen oder festen Zubereitung behandelt.

Die Verbindungen der Formel I können aber auch auf Samenkörner aufgebracht werden (Beizung/Coating), indem man die Körner entweder in einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel, Knospen oder Blätter.

Wirkstoffe der Formel I werden üblicherweise in Form von formulierten Zusammensetzungen verwendet und können gleichzeitig oder nacheinander mit weiteren Wirkstoffen auf die zu behandelnde Fläche oder Pflanze gegeben werden. Diese weiteren Wirkstoffe können auch andere in der Landwirtschaft angewendete Mittel umfassen, die in ihrer Nutzanwendung der Produktionssteigerung durch Förderung des Nutzpflanzenwachstums dienen, wie Düngemittel, Herbizide, Insektizide, Fungizide, Molluskizide u.a., oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden in unveränderter Form oder vorzugsweise zusammen mit den in der Formulierungstechnik üblichen Hilfsmitteln eingesetzt. Sie werden z.B. zu Emulsionskonzentraten, direkt versprühbaren oder verdünnbaren Lösungen, verdünnten Emulsionen, Spritzpulvern, löslichen Pulvern, Stäubemitteln, oder zu Granulaten durch Verkapselungen in z.B. polymeren Stoffen in bekannter Weise verarbeitet. Die Anwendungsverfahren wie Versprühen, Verstäuben, Verstreuen oder Giessen werden gleich wie die Art der Mittel den angestrebten Zielen und den gegebenen Verhältnissen entsprechend gewählt. Günstige Aufwandmengen liegen im allgemeinen bei 500 g bis 6 kg Aktivsubstanz (AS) je ha; bevorzugt 1 bis 4 kg AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenfalls mit oberflächenaktiven Substanzen (Tensiden).

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Substanzen kommen je nach Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na-oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. Das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979;
Dr. Helmut Stache "Tensid Taschenbuch", Carl Hanser Verlag München/Wien.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken.

1. Herstellungsbeispiele

Herstellungsbeispiel 1.1

Herstellung von 2,5-Bis(difluormethylthio)-1,3,4-thiadiazol

2 g Kaliumhydroxid werden in 24 ml Wasser gelöst. Zu dieser Lösung gibt man unter Rühren 9 g 2,5-Dimercapto-1,3,4-thiadiazol-Di-Kaliumsalz, 120 ml Dioxan, 0,4 g Kaliumjodid und 0,2 g Tetrabutylammoniumbromid. Anschliessend leitet man unter kräftigem Rühren bei 40 °C während 3 Stunden einen schwachen Strom Difluorchlormethan in die Lösung. Anschliessend dampft man die Lösungsmittel im Vakuum ab, nimmt den Rückstand in Methylenchlorid auf und wäscht die Lösung nacheinander mit Wasser, dann 1N Natronlauge aus und erhält nach dem Abdestillieren des Lösungsmittels 3,4 g des gewünschten Produktes, $n_D^{23}$ = 1,5452. Der Verteilungskoeffizient für Bodenapplikation ist logP = 1,7. Im Gegensatz dazu besitzt die aus dem Stand der Technik vorbekannte Verbindung V einen für Bodenapplikation sehr viel ungünstigeren Wert von logP = 5,0.

Analog dem vorstehenden Herstellungsbeispiel sowie der weiter oben beschriebenen Verfahren lassen sich folgende erfindungsgemässe Verbindungen herstellen. Die nachfolgend aufgeführten Verbindungen dienen der Illustration der vorliegenden Erfindung und stellen keine Begrenzung derselben dar.

Tabelle 1

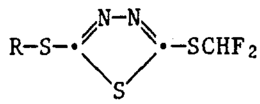

| Verb. Nr. | R | Physik. Daten |
|---|---|---|
| 1 | $CH_3-$ | $n_D^{25}$: 1,6067 |
| 2 | $C_2H_5$ | |
| 3 | $C_3H_7(n)-$ | |
| 4 | $C_3H_7(i)-$ | $n_D^{25}$: 1,5682 |
| 5 | $C_4H_9(n)-$ | |
| 6 | $ClCH_2-$ | |
| 7 | $F_2CH-$ | $n_D^{23}$: 1,5452 |
| 8 | $CF_3CF_2-$ | Oel |
| 9 | $CF_3CHFCF_2-$ | |
| 10 | $CF_2=CFCH_2CH_2-$ | $n_D^{25}$: 1,5278 |
| 11 | 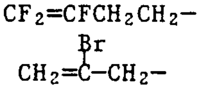 | Oel |
| 12 | $CH_2=CH-CH_2-$ | $n_D^{25}$: 1,5950 |
| 13 | $HC\equiv C-CH_2-$ | $n_D^{25}$: 1,6139 |
| 14 | 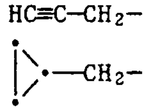 | Oel |
| 15 | 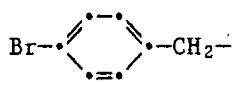 | Oel |
| 16 | 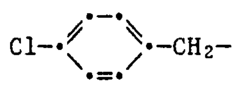 | $n_D^{25}$: 1,6293 |
| 17 | 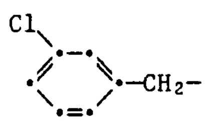 | $n_D^{25}$: 1,6259 |
| 18 | 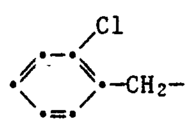 | $n_D^{25}$: 1,6355 |
| 19 | 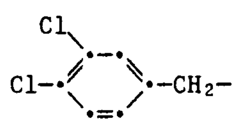 | $n_D^{25}$: 1,6351 |
| 20 | 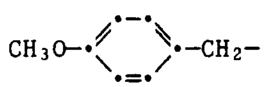 | halbfest |
| 21 | 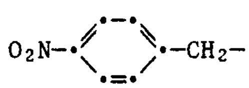 | |

Tabelle 1 (Fortsetzung)

| Verb. Nr. | R | Physik. Daten |
|---|---|---|
| 22 | 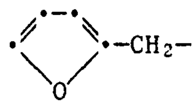 | $n_D^{25}$: 1,6138 |
| 23 | 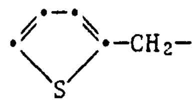 | $n_D^{25}$: 1,6503 |
| 24 | 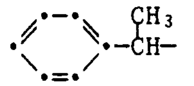 | $n_D^{23}$: 1,6100 |
| 25 | 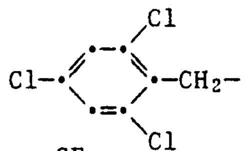 | |
| 26 | 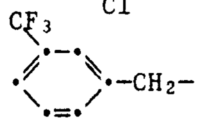 | $n_D^{25}$: 1,6554 |
| 27 | 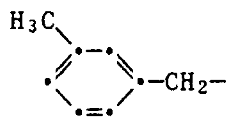 | |
| 28 | 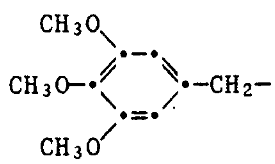 | halbfest |
| 29 | 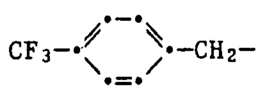 | Wachs |
| 30 | 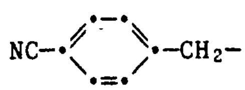 | Smp.: 57-59°C |
| 31 | 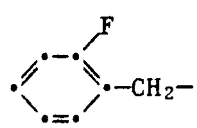 | $n_D^{25}$: 1,6059 |
| 32 | 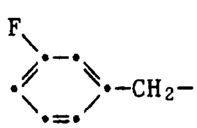 | $n_D^{25}$: 1,6061 |

Tabelle 1 (Fortsetzung)

| Verb. Nr. | R | Physik. Daten |
|---|---|---|
| 33 | 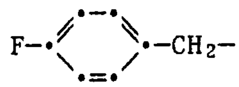 | $n_D^{25}$: 1,6620 |
| 34 | 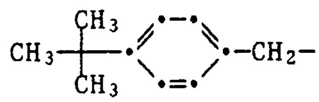 | $n_D^{25}$: 1,5875 |
| 35 | $CH_3OOCCH_2-$ | $n_D^{25}$: 1,5760 |
| 36 | $CH_3COCH_2-$ | Smp.: 48-50°C |
| 37 | 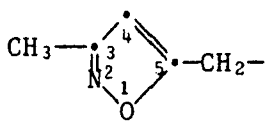 | halbfest |
| 38 | 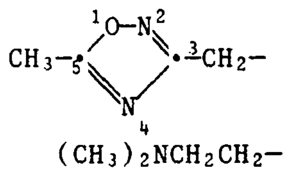 | $n_D^{25}$: 1,5905 |
| 39 | $(CH_3)_2NCH_2CH_2-$ | Oel |
| 40 | $CH_3OCH_2CH_2-$ | $n_D^{25}$: 1,5687 |
| 41 | $HOCH_2CH_2OCH_2CH_2-$ | |
| 42 | $CH_3CH_2OCH_2CH_2-$ | |
| 43 | $ClCH_2CH_2-$ | $n_D^{25}$: 1,6023 |
| 44 | $CH_3OCH_2CH_2OCH_2CH_2-$ | $n_D^{25}$: 1,5500 |
| 45 | $CH_3CH_2OCH_2CH_2-$ | |
| 46 | $CH_3CH_2OCH_2CH_2OCH_2CH_2-$ | |
| 47 | $CH_3CH_2OCH_2CH_2OCH_2CH_2OCH_2CH_2-$ | |
| 48 | $n-C_4H_9OCH_2CH_2OCH_2CH_2OCH_2CH_2-$ | |
| 49 | 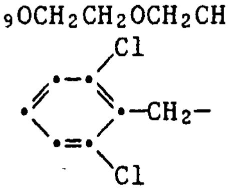 | $n_D^{25}$: 1,6353 |
| 50 | $CF_3CH_2-$ | |

2. Formulierungsbeispiele für flüssige Wirkstoffe der Formel I (% = Gewichtsprozent)

| 2.1 Emulsions-Konzentrate | a) | b) | c) |
|---|---|---|---|
| Wirkstoff aus den Tabellen 1-3 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenol-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2 Lösungen | a) | b) | c) | d) |
|---|---|---|---|---|
| Wirkstoff aus den Tabellen 1-3 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190 °C) | - | - | 94 % | - |
| (MG = Molekulargewicht) | | | | |

Die Lösungen sind zur Anwendung in Form kleinster Tropfen geeignet.

| 2.3 Granulate | a) | b) |
|---|---|---|
| Wirkstoff aus den Tabellen 1-3 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff aus den Tabellen 1-3 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| 2.5 Spritzpulver | a) | b) | c) |
|---|---|---|---|
| Wirkstoff aus den Tabellen 1-3 | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6 Emulsions-Konzentrat | |
|---|---|
| Wirkstoff aus den Tabellen 1-3 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.7 Stäubemittel | a) | b) |
|---|---|---|
| Wirkstoff aus den Tabellen 1-3 | 5 % | 8 % |
| Talkum | 95 % | - |
| Kaolin | - | 92 % |

Man erhält anwendungsfertige Stäubemittel, indem der Wirkstoff mit den Trägern vermischt und auf einer geeigneten Mühle vermahlen wird.

| 2.8 Extruder Granulat | |
|---|---|
| Wirkstoff aus den Tabellen 1-3 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9 Umhüllungs-Granulat | |
|---|---|
| Wirkstoff aus den Tabellen 1-3 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.10 Suspensions-Konzentrat | |
|---|---|
| Wirkstoff aus den Tabellen 1-3 | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

3.1 Wirkung gegen Meloidogyne incognita auf Tomaten

Eier von Meloidogyne incognita werden in Sand eingemischt. Dieses Gemisch wird dann in 200 ml fassende Tontöpfe eingebracht (5000 Eier pro Topf). Am gleichen Tag wird pro Topf eine 3 Wochen alte Tomatenpflanze gepflanzt und der formulierte Wirkstoff mittels Drenchapplikation in die Töpfe hineingegeben (0,0006 % Aktivsubstanz bezogen auf das Bodenvolumen). Die eingetopften Pflanzen werden nun bei einer Temperatur von 26±1°C und einer relativen Luftfeuchtigkeit von 60 % in einem Gewächshaus aufgestellt. Nach Ablauf von 4 Wochen erfolgt eine Evaluierung durch Untersuchung der Pflanzen auf Wurzelgallbildung nach dem sogenannten Wurzelgallen-Index („Root-Knot Index").

Verbindungen aus der Tabelle 1 zeigen gegen Meloidogyne incognita durch weitgehende Reduktion der Wurzelgallbildung gute Wirksamkeit. Unbehandelte jedoch infizierte Kontrollpflanzen weisen dagegen starke Wurzelgallbildung auf (= 100 %). So hemmt z.B. die Verbindung Nr. 7 im obigen Versuch die Wurzelgallbildung fast vollständig (0-10 % Restbefall).

**Patentansprüche**
**Patentansprüche für folgende Vertragsstaaten : AT, BE, CH, DE, FR, GB, GR, IT, LI, LU, NL**

**1.** 2-Thio-5-difluormethylthio-1,3,4-thiadiazole der Formel I

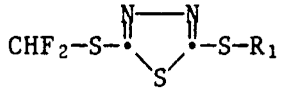

(I)

worin
$R_1$ unsubstituiertes oder durch Halogen, Phenyl, $C_1$-$C_3$-Alkoxy oder Hydroxy substituiertes $C_1$-$C_{12}$-Alkyl oder durch Carbonyl, O-Alkylcarboxyl, Amino oder durch einen Heterocyclus substituiertes $C_1$-$C_3$-Alkyl, unsubstituiertes oder durch Halogen substituiertes $C_3$-$C_7$-Cycloalkyl oder unsubstituiertes Cyclopropylmethyl, unsubstituiertes oder durch Halogen substituiertes $C_3$-$C_7$-Alkenyl, unsubstituiertes oder durch Halogen substituiertes $C_3$-$C_7$-Alkinyl, unsubstituiertes oder durch $C_1$-$C_4$-Alkyl, Halogen, Nitro, Cyano oder Trifluormethyl ein-, zwei- oder dreifach kernsubstituiertes Benzyl oder den Rest $R_2$-(O-$CH_2$-$CH_2$-$)_n$, bedeutet, worin $R_2$ für Wasserstoff, oder für unsubstituiertes oder durch Hydroxy substituiertes $C_1$-$C_4$-Alkyl und n für die Zahlen 1-3 stehen.

**2.** 2-Thio-5-difluormethylthio-1,3,4-thiadiazole der Formel I gemäss Anspruch 1,
worin
$R_1$ unsubstituiertes oder durch Halogen, Phenyl, $C_1$-$C_3$-Alkoxy oder Hydroxy substituiertes $C_1$-$C_4$-Alkyl, $C_3$-$C_4$-Alkenyl, $C_3$-$C_4$-Alkinyl, ferner den Carbomethoxy-methyl-, den Acetonyl-, den 2-Dimethylamino-äthyl-, den 2-Furanylmethyl-, den 2-Thienylmethyl-, den 3-Methyl-oxazol-5-yl-methyl-oder den 5-Methyl-1,2,4-oxadiazol-3-yl-methylrest oder den Rest

$$R_2-(OCH_2CH_2)_n-$$

bedeutet.

**3.** 2-Thio-5-difluormethylthio-1,3,4-thiadiazole gemäss Anspruch 1, worin
$R_1$ unsubstituiertes oder durch Halogen substituiertes $C_1$-$C_3$-Alkyl, ferner $C_3$-Alkenyl, $C_3$-Alkinyl und unsubstituiertes oder durch Halogen ein-oder zweifach kernsubstituiertes Benzyl bedeutet.

**4.** Das 2,5-Bis(difluormethylthio)-1,3,4-thiadiazol.

**5.** Verfahren zur Herstellung der Verbindungen der Formel I gemäss Anspruch 1, dadurch gekennzeichnet, dass man

a) das 2,5-Dimercapto-1,3,4-thiadiazol der Formel II in einem Lösungsmittel zunächst mit einer Halogenverbindung der Formel III, worin $R_1$ die im Anspruch 1 angegebene Bedeutung hat und X für Chlor oder Brom steht, in Gegenwart einer Base zum 5-Mercapto-1,3,4-thiadiazol-Derivat der Formel IV umsetzt, dann das resultierende Zwischenprodukt der Formel IV in einem Lösungsmittel mit Difluorchlormethan in Gegenwart einer Base in das Endprodukt der Formel I überführt:

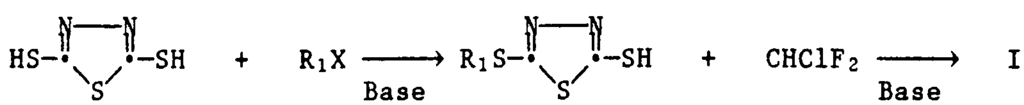


II III IV

b) eine Verbindung der Formel VI in einem Lösungsmittel mit Difluorchlormethan zum Endprodukt der Formel I umsetzt:

$$K_2(S_2C{=}N{-}NH{-}CS_2R) + 2CHClF_2 \longrightarrow I.$$

VI

**6.** Schädlingsbekämpfungsmittel zur Bekämpfung oder Verhütung des Befalls von Pflanzen durch Nematoden, dadurch gekennzeichnet, dass es als aktive Komponente mindestens eine Verbindung der Formel I, gemäss Anspruch 1 enthält.

**7.** Mittel gemäss Anspruch 6, dadurch gekennzeichnet, dass es als aktive Komponente mindestens eine Verbindung der Formel I gemäss der Ansprüche 2 und 3 enthält.

**8.** Mittel gemäss Anspruch 7, dadurch gekennzeichnet, dass es als aktive Komponente 2,5-Bis-(difluormethylthio)-1,3,4-thiadiazol enthält.

**9.** Mittel nach einem der Ansprüche 6 bis 8, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

**10.** Mittel nach Anspruch 9, dadurch gekennzeichnet, dass es 0,1 bis 95 % eines Wirkstoffes der Formel I, 99,8 bis 5 % eines festen oder flüssigen Zusatzstoffes und 0,1 bis 25 % eines Tensides enthält.

**11.** Verfahren zur Herstellung eines agrochemischen Mittels, dadurch gekennzeichnet, dass mindestens eine Verbindung der Formel I gemäss Anspruch 1 mit geeigneten festen oder flüssigen Zusatzstoffen und Tensiden innig vermischt.

**12.** Verfahren zur Bekämpfung oder Verhütung eines Befalls von Kulturpflanzen durch Nematoden, dadurch gekennzeichnet, dass man eine Verbindung der Formel I gemäss einem der Ansprüche 1 bis 4 auf die Pflanze oder deren Standort appliziert.

**13.** Verwendung von Verbindungen der Formel I gemäss Anspruch 1 zur Bekämpfung und/oder zur präventiven Verhütung eines Befalls von Pflanzen durch Nematoden.

**14.** Verwendung gemäss Anspruch 13, von Verbindungen der Formel I gemäss einem der Ansprüche 2 bis 4.

**15.** Verwendung gemäss Anspruch 13, dadurch gekennzeichnet, dass es sich bei den Nematoden um pflanzenparasitäre Arten handelt.

**16.** Verwendung gemäss Anspruch 15 gegen Nematoden der Gattung Meloidogyne, Heterodera oder Globodera.

**Patentansprüche für folgenden Vertragsstaat : ES**

**1.** Verfahren zur Herstellung von 2-Thio-5-difluormethylthio-1,3,4-thiadiazolender Formel I

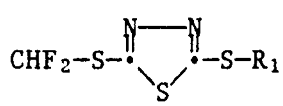

(I)

worin
$R_1$ unsubstituiertes oder durch Halogen, Phenyl, $C_1$-$C_3$-Alkoxy oder Hydroxy substituiertes $C_1$-$C_{12}$-Alkyl oder durch Carbonyl, O-Alkylcarboxyl, Amino oder durch einen Heterocyclus substituiertes $C_1$-$C_3$-Alkyl, unsubstituiertes oder durch Halogen substituiertes $C_3$-$C_7$-Cycloalkyl oder unsubstituiertes Cyclopropylmethyl, unsubstituiertes oder durch Halogen substituiertes $C_3$-$C_7$-Alkenyl, unsubstituiertes oder durch Halogen substituiertes $C_3$-$C_7$-Alkinyl, unsubstituiertes oder durch $C_1$-$C_4$-Alkyl, Halogen, Nitro, Cyano oder Trifluormethyl ein-, zwei- oder dreifach kernsubstituiertes Benzyl oder den Rest $R_2$-(O-$CH_2$-$CH_2$-$)_n$, bedeutet, worin $R_2$ für Wasserstoff, oder für unsubstituiertes oder durch Hydroxy substituiertes $C_1$-$C_4$-Alkyl und n für die Zahlen 1-3 stehen, dadurch gekennzeichnet, dass man

a) das 2,5-Dimercapto-1,3,4-thiadiazol der Formel II in einem Lösungsmittel zunächst mit einer Halogenverbindung der Formel III, worin $R_1$ die im Anspruch 1 angegebene Bedeutung hat und X für Chlor oder Brom steht, in Gegenwart einer Base zum 5-Mercapto-1,3,4-thiadiazol-Derivat der Formel IV umsetzt, dann das resultierende Zwischenprodukt der Formel IV in einem Lösungsmittel mit Difluorchlormethan in Gegenwart einer Base in das Endprodukt der Formel I überführt:

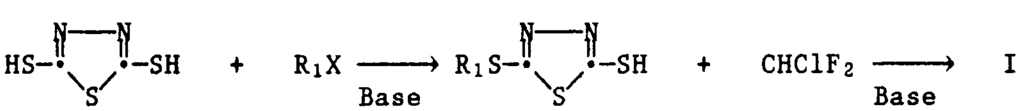


II III IV

b) eine Verbindung der Formel VI in einem Lösungsmittel mit Difluorchlormethan zum Endprodukt der Formel I umsetzt:

$$K_2(S_2C{=}N\text{-}NH\text{-}CS_2R) + 2CHClF_2 \longrightarrow I.$$

VI

**2.** Verfahren gemäss Anspruch 1, dadurch gekennzeichnet, dass Verbindungen hergestellt werden, worin $R_1$ unsubstituiertes oder durch Halogen, Phenyl, $C_1$-$C_3$-Alkoxy oder Hydroxy substituiertes $C_1$-$C_4$-Alkyl,

$C_3$-$C_4$-Alkenyl, $C_3$-$C_4$-Alkinyl, ferner den Carbomethoxy-methyl-, den Acetonyl-, den 2-Dimethylamino-äthyl-, den 2-Furanylmethyl-, den 2-Thienylmethyl-, den 3-Methyl-oxazol-5-yl-methyl-oder den 5-Methyl-1,2,4-oxadiazol-3-yl-methylrest oder den Rest bedeutet.

**3.** Verfahren gemäss Anspruch 1, dadurch gekennzeichnet, dass Verbindungen hergestellt werden, worin $R_1$ unsubstituiertes oder durch Halogen substituiertes $C_1$-$C_3$-Alkyl, ferner $C_3$-Alkenyl, $C_3$-Alkinyl und unsubstituiertes oder durch Halogen ein-oder zweifach kernsubstituiertes Benzyl bedeutet.

**4.** Verfahren gemäss Anspruch 3, dadurch gekennzeichnet, dass das 2,5-Bis(difluormethylthio)-1,3,4-thiadiazol hergestellt wird.

**Claims**
**Claims for the following Contracting States: AT, BE, CH, DE, FR, GB, GR, IT, LI, LU, NL**

**1.** A 2-thio-5-difluoromethylthio-1,3,4-thiadiazole of formula I

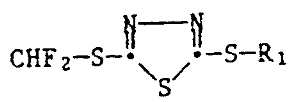

(I)

wherein $R_1$ is $C_1$-$C_{12}$alkyl that is unsubstituted or substituted by halogen, phenyl, $C_1$-$C_3$alkoxy or by hydroxy, $C_1$-$C_3$alkyl that is substituted by carbonyl, O-alkylcarboxy, amino or by a heterocycle, unsubstituted or halo-substituted $C_3$-$C_7$cycloalkyl or unsubstituted cyclopropylmethyl, unsubstituted or halo-substituted $C_3$-$C_7$alkenyl, unsubstituted or halo-substituted $C_3$-$C_7$-alkynyl, benzyl that is unsubstituted or mono-, di- or tri-substituted in the nucleus by $C_1$-$C_4$alkyl, halogen, nitro, cyano or by trifluoromethyl, or is the radical $R_2$-(O-$CH_2$-$CH_2$-$)_n$ wherein $R_2$ is hydrogen or unsubstituted or hydroxy-substituted $C_1$-$C_4$alkyl and n is a number from 1 to 3.

**2.** A 2-thio-5-difluoromethylthio-1,3,4-thiadiazole of formula I according to claim 1, wherein $R_1$ is $C_1$-$C_4$alkyl that is unsubstituted or substituted by halogen, phenyl, $C_1$-$C_3$alkoxy or by hydroxy, $C_3$-$C_4$alkenyl, $C_3$-$C_4$alkynyl, or the methoxycarbonylmethyl, the acetonyl, the 2-dimethylaminoethyl, the 2-furanylmethyl, the 2-thienylmethyl, the 3-methyloxazol-5-ylmethyl, the 5-methyl-1,2,4-oxadiazol-3-ylmethyl radical or the radical $R_2$-$(OCH_2CH_2)_n$-.

**3.** A 2-thio-5-difluoromethylthio-1,3,4-thiadiazole according to claim 1, wherein $R_1$ is unsubstituted or halo-substituted $C_1$-$C_3$alkyl, or is $C_3$alkenyl or $C_3$alkynyl, or benzyl that is unsubstituted or mono- or di-substituted in the nucleus by halogen.

**4.** 2,5-bis(difluoromethylthio)-1,3,4-thiadiazole.

**5.** A process for the preparation of a compound of formula I according to claim 1, which comprises
a) reacting the 2,5-dimercapto-1,3,4-thiadiazole of formula II in a solvent first with a halogen compound of formula III, wherein $R_1$ is as defined in claim 1 and X is chlorine or bromine, in the presence of a base to form the 5-mercapto-1,3,4-thiadiazole derivative of formula IV, then converting the resulting intermediate of formula IV in a solvent with difluorochloromethane in the presence of a base into the end product of formula I:

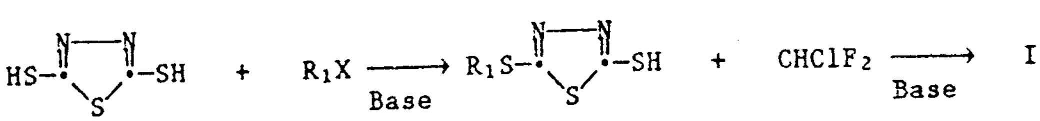


II III IV

or

b) reacting a compound of formula VI in a solvent with difluorochloromethane to form the end product of formula I:

$$\underset{\text{VI}}{K_2(S_2C{=}N{-}NH{-}CS_2R)} + 2CHClF_2 \longrightarrow \text{I.}$$

**6.** A pesticidal composition for controlling or preventing attacks on plants by nematodes, which comprises as active ingredient at least one compound of formula I according to claim 1.

**7.** A composition according to claim 6, which comprises as active ingredient at least one compound of formula I according to claims 2 and 3.

**8.** A composition according to claim 7, which comprises 2,5-bis(difluoromethylthio)-1,3,4-thiadiazole as active ingredient.

**9.** A composition according to any one of claims 6 to 8, which comprises 0.1 to 99 % of a compound of formula I, 99.9 to 1 % of a solid or liquid adjuvant and 0 to 25 % of a surfactant.

**10.** A composition according to claim 9, which comprises 0.1 to 95 % of a compound of formula I, 99.8 to 5 % of a solid or liquid adjuvant and 0.1 to 25 % of a surfactant.

**11.** A process for the preparation of an agrochemical composition, which comprises homogeneously mixing at least one compound of formula I according to claim 1 with suitable solid or liquid adjuvants and surfactants.

**12.** A method of controlling or preventing attacks on cultivated plants by nematodes, which comprises applying a compound of formula I according to any one of claims 1 to 4 to the plant or the locus thereof.

**13.** The use of a compound of formula I according to claim 1 for controlling and/or preventing attacks on plants by nematodes.

**14.** The use according to claim 13 of a compound of formula I according to any one of claims 2 to 4.

**15.** The use according to claim 13, wherein the nematodes are species that are parasites of plants.

**16.** The use according to claim 15 against nematodes of the genus Meloidogyne, Heterodera or Globodera.

**Claims for the following Contracting State : ES**

**1.** A process for the preparation of a 2-thio-5-difluoromethylthio-1,3,4-thiadiazole of formula I

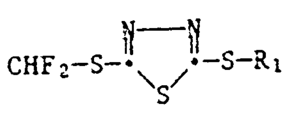

(I)

wherein $R_1$ is $C_1$-$C_{12}$alkyl that is unsubstituted or substituted by halogen, phenyl, $C_1$-$C_3$alkoxy or by hydroxy, $C_1$-$C_3$alkyl that is substituted by carbonyl, O-alkylcarboxy, amino or by a heterocycle, unsubstituted or halo-substituted $C_3$-$C_7$cycloalkyl or unsubstituted cyclopropylmethyl, unsubstituted or halo-substituted $C_3$-$C_7$alkenyl, unsubstituted or halo-substituted $C_3$-$C_7$alkynyl, benzyl that is unsubstituted or mono-, di- or tri-substituted in the nucleus by $C_1$-$C_4$alkyl, halogen, nitro, cyano or by

trifluoromethyl, or is the radical $R_2$-(O-$CH_2$-$CH_2$-$)_n$ wherein $R_2$ is hydrogen or unsubstituted or hydroxy-substituted $C_1$-$C_4$alkyl and n is a number from 1 to 3, which comprises

a) reacting the 2,5-dimercapto-1,3,4-thiadiazole of formula II in a solvent first with a halogen compound of formula III, wherein $R_1$ is as defined in claim 1 and X is chlorine or bromine, in the presence of a base to form the 5-mercapto-1,3,4-thiadiazole derivative of formula IV, then converting the resulting intermediate of formula IV in a solvent with difluorochloromethane in the presence of a base into the end product of formula I:

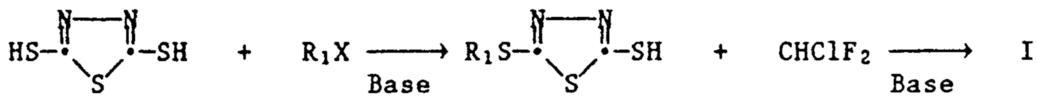


II III IV

or

b) reacting a compound of formula VI in a solvent with difluorochloromethane to form the end product of formula I:

$$K_2(S_2C{=}N\text{-}NH\text{-}CS_2R) + 2CHClF_2 \longrightarrow I.$$

VI

**2.** A process according to claim 1, which comprises preparing a compound wherein $R_1$ is $C_1$-$C_4$alkyl that is unsubstituted or substituted by halogen, phenyl, $C_1$-$C_3$alkoxy or by hydroxy, $C_3$-$C_4$alkenyl, $C_3$-$C_4$alkynyl, or the methoxycarbonylmethyl, the acetonyl, the 2-dimethylaminoethyl, the 2-furanylmethyl, the 2-thienylmethyl, the 3-methyloxazol-5-ylmethyl, the 5-methyl-1,2,4-oxadiazol-3-ylmethyl radical or the radical $R_2$-($OCH_2CH_2$$)_n$-.

**3.** A process according to claim 1, which comprises preparing a compound wherein $R_1$ is unsubstituted or halo-substituted $C_1$-$C_3$alkyl, or is $C_3$alkenyl or $C_3$alkynyl, or benzyl that is unsubstituted or mono- or di-substituted in the nucleus by halogen.

**4.** A process according to claim 3, which comprises preparing 2,5-bis(difluoromethylthio)-1,3,4-thiadiazole.

**Revendications**

**Revendications pour les Etats contractants suivants : AT, BE, CH, DE, FR, GB, GR, IT, LI, LU, NL**

**1.** 2-thio-5-difluorométhylthio-1,3,4-thiadiazoles de formule I

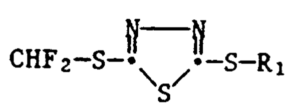

(I)

dans laquelle

$R_1$ représente un groupe alkyle en C 1-C 12 non substitué ou substitué par des halogènes, des groupes phényle, alcoxy en C 1-C 3 ou hydroxy, ou un groupe alkyle en C 1-C 3 substitué par des groupes carbonyle, O-alkylcarboxyle, amino ou par un hétérocycle, un groupe cycloalkyle en C 3-C 7 non substitué ou substitué par des halogènes, ou un groupe cyclopropylméthyle non substitué, un groupe alcényle en C 3-C 7 non substitué ou substitué par des halogènes, un groupe alcynyle en C 3-C 7 non substitué ou substitué par des halogènes, un groupe benzyle non substitué ou substitué une, deux ou trois fois dans le noyau par des groupes alkyle en C 1-C 4, des halogènes, des groupes nitro, cyano ou trifluorométhyle, ou le groupe $R_2$-(O-$CH_2CH_2$$)_n$ dans lequel $R_2$ représente l'hydrogène ou un groupe alkyle en C 1-C 4 non substitué ou substitué par des groupes hydroxy, et n a une valeur de 1 à 3.

**2.** 2-thio-5-difluorométhylthio-1,3,4-thiadiazoles de formule I de la revendication 1, dans laquelle $R_1$ représente un groupe alkyle en C 1-C 4 non substitué ou substitué par des halogènes, des groupes phényle, alcoxy en C 1-C 3 ou hydroxy, un groupe alcényle en C 3-C 4, un groupe alcynyle en C 3-C 4, ou encore un groupe carbométhoxyméthyle, acétonyle, 2-diméthylaminoéthyle, 2-furannylméthyle, 2-thiénylméthyle, 3-méthyloxazole-5-ylméthyle ou 5-méthyl-1,2,4-oxadiazole-3-yle, ou le groupe $R_2-(O-CH_2-CH_2)_n$.

**3.** 2-thio-5-difluorométhylthio-1,3,4-thiadiazoles selon revendication 1, dans lesquels $R_1$ représente un groupe alkyle en C 1-C 3 non substitué ou substitué par des halogènes, ou encore un groupe alcényle en C 3, alcynyle en C 3 ou un groupe benzyle non substitué ou mono- ou di-substitué dans le noyau par des halogènes.

**4.** Le 2,5-bis-(difluorométhylthio)-1,3,4-thiadiazole.

**5.** Procédé de préparation des composés de formule I de la revendication 1, caractérisé en ce que :

a) on fait réagir le 2,5-dimercapto-1,3,4-thiadiazole de formule II, dans un solvant, d'abord avec un dérivé halogéné de formule III dans laquelle $R_1$ a les significations indiquées dans la revendication 1 et X représente le chlore ou le brome, en présence d'une base, la réaction donnant le dérivé de 5-mercapto-1,3,4-thiadiazole de formule IV qu'on convertit dans un solvant, à l'aide du difluorochlorométhane, en présence d'une base, en le produit final de formule I :

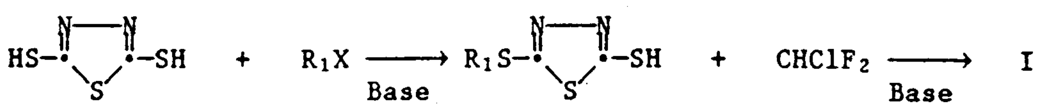


II III IV

b) on fait réagir un composé de formule VI, dans un solvant, avec le difluorochlorométhane, la réaction donnant le produit final de formule I :

$$K_2(S_2C=N-NH-CS_2R) + 2CHClF_2 \longrightarrow I.$$

VI

**6.** Produit pesticide servant à combattre ou a prévenir une attaque de végétaux par les nématodes, caractérisé en ce qu'il contient au moins un composé de formule I de la revendication 1 en tant que composant actif.

**7.** Produit selon la revendication 6, caractérisé en ce qu'il contient au moins un composé de formule I selon les revendications 2 et 3 en tant que composant actif.

**8.** Produit selon la revendication 7, caractérisé en ce qu'il contient en tant que composant actif le 2,5-bis-(difluorométhylthio)-1,3,4-thiadiazole.

**9.** Produit selon l'une des revendications 6 à 8, caractérisé en ce qu'il contient de 0,1 à 99% d'une substance active de formule I, de 99,9 à 1% d'un additif solide ou liquide et de 0 à 25% d'un agent tensioactif.

**10.** Produit selon la revendication 9, caractérisé en ce qu'il contient de 0,1 à 95% d'une substance active de formule I, de 99,8 à 5% d'un additif solide ou liquide et de 0,1 à 25% d'un agent tensioactif.

**11.** Procédé pour préparer un produit agrochimique, caractérisé en ce que l'on mélange au moins un composé de formule I de la revendication 1 avec des additifs solides ou liquides appropriés et des

agents tensioactifs.

**12.** Procédé pour combattre ou prévenir une attaque de végétaux cultivés par les nématodes, caractérisé en ce que l'on applique un composé de formule I selon l'une des revendications 1 à 4 sur les végétaux ou leur lieu de plantation.

**13.** Utilisation des composés de formule I selon revendication 1 pour combattre et/ou prévenir une attaque de végétaux par des nématodes.

**14.** Utilisation selon revendication 13, de composés de formule I selon l'une des revendications 2 à 4.

**15.** Utilisation selon revendication 13, caractérisée en ce que les nématodes appartiennent à des espèces parasitaires des végétaux.

**16.** Utilisation selon revendication 15, sur des nématodes des genres Meloidogyne, Heterodera ou Globodera.

**Revendications pour l'Etat contractant suivant : ES**

**1.** Procédé de préparation des 2-thio-5-difluorométhylthio-1,3,4-thiadiazoles de formule I

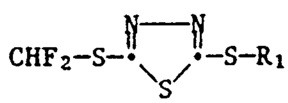

(I)

dans laquelle
$R_1$ représente un- groupe alkyle en C 1-C 12 non substitué ou substitué par des halogènes, des groupes phényle, alcoxy en C 1-C 3 ou hydroxy, ou un groupe alkyle en C 1-C 3 substitué par des groupes carbonyle, O-alkylcarboxyle, amino ou par un hétérocycle, un groupe cycloalkyle en C 3-C 7 non substitué ou substitué par des halogènes, ou un groupe cyclopropylméthyle non substitué, un groupe alcényle en C 3-C 7 non substitué ou substitué par des halogènes, un groupe alcynyle en C 3-C 7 non substitué ou substitué par des halogènes, un groupe benzyle non substitué ou substitué une, deux ou trois fois dans le noyau par des groupes alkyle en C 1-C 4, des halogènes, des groupes nitro, cyano ou trifluorométhyle, ou le groupe $R_2$-(O-$CH_2$-$CH_2$)$_n$ dans lequel $R_2$ représente l'hydrogène ou un groupe alkyle en C 1-C 4 non substitué ou substitué par des groupes hydroxy et n a une valeur de 1 à 3, caractérisé en ce que :

a) on fait réagir le 2,5-dimercapto-1,3,4-thiadiazole de formule II, dans un solvant, d'abord avec un dérivé halogéné de formule III dans laquelle $R_1$ a les significations indiquées dans la revendication 1 et X représente le chlore ou le brome, en présence d'une base, la réaction donnant le dérivé de 5-mercapto-1,3,4-thiadiazole de formule IV qu'on convertit dans un solvant, à l'aide du difluorochlorométhane, en présence d'une base, en le produit final de formule I

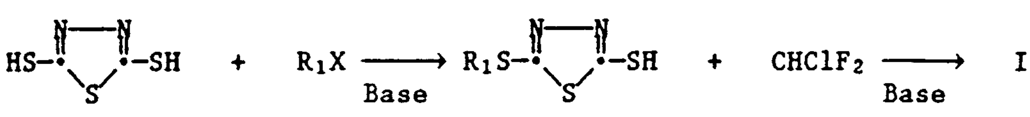


II III IV

b) on fait réagir un composé de formule VI, dans un solvant, avec le difluorochlorométhane, ce qui donne le produit final de formule I

$$K_2(S_2C{=}N\text{-}NH\text{-}CS_2R) + 2CHClF_2 \longrightarrow I.$$

VI

2. Procédé selon la revendication 1, caractérise en ce que l'on prépare des composés dans lesquels $R_1$ représente un groupe alkyle en C 1-C 4 non substitué ou substitué par des halogènes, des groupes phényle, alcoxy en C 1-C 3 ou hydroxy, un groupe alcényle en C 3-C 4, alcynyle en C 3-C 4, ou encore un groupe carbométhoxyméthyle, acétonyle, 2-diméthylaminoéthyle, 2-furannylméthyle, 2-thiénylméthyle, 3-méthyloxazole-5-ylméthyle ou 5-méthyl-1,2,4-oxadiazole-3-yle, ou le groupe $R_2$-$(OCH_2CH_2)_n$.

3. Procédé selon la revendication 1, caractérisé en ce que l'on prépare des composés dans lesquels $R_1$ représente un groupe alkyle en C 1-C 3 non substitué ou substitué par des halogènes, ou encore alcényle en C 3, alcynyle en C 3 ou un groupe benzyle non substitué ou mono- ou di-substitué dans le noyau par des halogènes.

4. Procédé selon la revendication 3, caractérisé en ce que l'on prépare le 2,5-bis-(difluorométhylthio)-1,3,4-thiadiazole.